# EUROPÄISCHE PATENTSCHRIFT

(11) **EP 4 190 265 B1**
(45) Veröffentlichungstag und Bekanntmachung des Hinweises auf die Patenterteilung: **15.04.2026**
(21) Anmeldenummer: 21211739.4
(22) Anmeldetag: 01.12.2021
(51) Int. Cl.: A61B 34/20, A61B 18/00, A61B 90/00, A61B 90/50

(54) **VORRICHTUNG UND VERFAHREN ZUR BILDGESTÜTZTEN UNTERSTÜTZUNG EINES ANWENDERS**
DEVICE AND METHOD FOR IMAGE-AIDED SUPPORT OF A USER
DISPOSITIF ET PROCÉDÉ D'AIDE ASSISTÉE PAR IMAGE À UN UTILISATEUR

(43) Veröffentlichungstag der Anmeldung: 07.06.2023
(73) Patentinhaber: Erbe Elektromedizin GmbH, 72072 Tübingen (DE)
(72) Erfinder: ZENKER, Matthias, 72074 Tübingen (DE); DANIEL, Yannick, 72336 Balingen (DE); JURJUT, Ovidiu, 72070 Tübingen (DE)
(74) Vertreter: Rüger Abel Patentanwälte PartGmbB

(56) Entgegenhaltungen:
- EP-A1- 3 718 496
- EP-B1- 3 346 807
- WO-A1-2015/085011
- WO-A1-2017/160808
- RU-C1- 2 582 213
- US-A1- 2011 082 451

## Beschreibung

Die Erfindung betrifft eine Vorrichtung sowie ein Verfahren zur bildgestützten Unterstützung eines Anwenders eines (medizinischen) Instruments, das dazu eingerichtet ist, ein Behandlungsmedium auf eine Gewebeoberfläche zu applizieren.

Instrumente, die es einem Anwender erlauben, ein Behandlungsmedium, wie beispielsweise ein niederthermisches Plasma, frei Hand und kleinflächig auf eine zu behandelnde Gewebeoberfläche zu applizieren, sind grundsätzlich bekannt. Es werden hierbei beispielsweise Gewebeoberflächen, wie Haut, Schleimhaut, oder Wunden, von dem Anwender behandelt, indem ein Behandlungsmedium, wie beispielsweise ein Kaltplasma (nichtthermisches Plasma) oder ein niederenergetisches Argonplasma, auf die Gewebeoberfläche appliziert wird.

Diese Behandlung der Gewebeoberfläche führt zu keinem oder nur geringem thermischen Effekt im Gewebe, wodurch keine Koagulation oder Karbonisation des Gewebes auftritt. Die lokale Temperatur auf der Gewebeoberfläche bleibt unterhalb der Denaturierungstemperatur von Proteinen (ca. 60-70°C). Die behandelte Gewebeoberfläche verändert sich optisch nicht, weshalb der Anwender nicht erkennen kann, in welchem Bereich der Gewebeoberfläche bereits das Behandlungsmedium appliziert wurde. Auch ist für den Anwender nicht erkennbar, welche Dosierung oder welche Einwirkdauer die einzelnen Stellen des zu behandelnden Bereichs erhalten haben.

So muss sich der Anwender also möglichst genau merken, in welchen Bereichen das Behandlungsmedium für welche Zeitdauer appliziert wurde.

Dies stellt eine erhöhte Anforderung an die Konzentration des Anwenders. Außerdem unterliegt die Einschätzung der Zeitdauer, in der das Behandlungsmedium an einer bestimmten Stelle appliziert wurde, dem subjektiven Empfinden des Anwenders. Außerdem kann der Anwender wegen des Ausbleibens sichtbarer Auswirkungen der Behandlung auf das Gewebe dazu verführt werden, schon behandelte Partien wieder und wieder zu überstreichen und so eine Überdosierung vorzunehmen.

Genauso kann es allerdings vorkommen, dass der Anwender einzelne Bereiche überhaupt nicht oder nur zu kurz behandelt und diese somit eine Unterdosierung erfahren.

Dies führt dazu, dass die Behandlungsqualität und die Gleichmäßigkeit, mit der das Behandlungsmedium auf die zu behandelnde Gewebeoberfläche aufgetragen wird, mitunter stark schwanken können. Auch sind hierdurch Behandlungsergebnisse nur schwer reproduzierbar und können nicht objektiv überprüft und/oder dokumentiert werden.

Es gibt Ansätze, bei denen versucht wird, die Gleichmäßigkeit, mit der das Behandlungsmedium auf die Gewebeoberfläche aufgetragen wird, durch neu entwickelte, großflächige Plasmaquellen zu verbessern. In der DE 10 2014 220 488 A1 wird beispielsweise eine großflächige Plasmaquelle für kaltes Atmosphärendruckplasma vorgeschlagen, mit der eine relativ große Fläche (wie zum Beispiel eine Fläche von etwa 100 cm²) behandelt werden kann. Insbesondere bei zu behandelnden Bereichen, die keine einfachen Formen (wie beispielsweise rechteckig, quadratisch, elliptisch oder kreisförmig) sondern komplexe und/oder unregelmäßige Formen aufweisen, kann es dazu kommen, dass unbeabsichtigt Stellen behandelt werden, die außerhalb des zu behandelnden Bereichs liegen.

Weitere bekannte Ansätze schlagen eine Automatisierung des Auftragens des Behandlungsmediums vor. Beispielsweise wird in der DE 10 2010 011 643 A1 eine Plasmaquelle beschrieben, die mit einer von einer Steuereinrichtung gesteuerten Bewegungseinrichtung relativ zur Gewebeoberfläche automatisiert bewegt wird. Dies greift jedoch direkt in den Behandlungsprozess ein. Darüber hinaus ist eine vollständige Automatisierung des Auftragens entwicklungs- und damit kostenintensiv.

In *"*Concept for Improved Handling Ensures Effective Contactless Plasma Treatment of Patients with kINPen® MED", Applied Sciences, Bd. 10, Nr. 17, Art. Nr. 17, Jan. 2020, doi: 10.3390/app10176133 wird ein Sensorsystem vorgeschlagen, das den Abstand des Instruments zum Behandlungsgebiet bestimmt und den Bereich ausleuchtet, in welchem das Plasma voraussichtlich aufgetragen wird. Dies wird dem Anwender jedoch lediglich für den aktuellen Zeitpunkt angezeigt. Der Anwender muss sich somit weiterhin merken, in welchen Bereichen das Behandlungsmedium für welche Zeitdauer appliziert wurde.

Ferner offenbart die WO 2011/044248 A2 bzw. US 2011/082451 A1 ein System, bei dem bei einer Ablationsoperation mittels einer Videokamera das Operationsgebiet beobachtet wird, wobei die Qualität der bei der Ablation im Gewebe erzeugten Läsionen von einem Prozessor ausgewertet wird. Entsprechend der bestimmten Qualität werden Farbmarker in die Live-Bilder des Operationsgebiets eingeblendet.

In der DE 10 2015 100 927 A1 wird eine Assistenzeinrichtung zur bildgebenden Unterstützung eines Operateurs während eines chirurgischen Eingriffs beschrieben. Die Assistenzeinrichtung umfasst eine Kamera und eine Bildverarbeitungseinheit, wobei die Bildverarbeitungseinheit dazu eingerichtet ist, das verwendete medizinische Instrument ohne die Notwendigkeit von auf dem Instrument angebrachten Markern zu erkennen. Darüber hinaus beschreibt die EP 3 718 496 A1 eine Vorrichtung, bei der einer Sonde, die mit Gewebe in einem Körperhohlraum in Kontakt steht, Energie zugeführt wird, um das Gewebe abzutragen. Während des Energieeintrags werden Signale von einem Positionsgeber in der Sonde empfangen, die einen Hinweis auf die Position der Sonde in der Kavität geben. Die Signale werden verarbeitet, um 3D-Positionskoordinatenpunkte abzuleiten, die der Position der Sonde zu einer Reihe von Zeitpunkten entsprechen, zu denen die Energie eingebracht wurde. Die RU 2 582 213 C1 beschreibt ein laserchirurgisches System, bestehend aus einem CO₂-Laser mit Pilotstrahlführung, einer Videoüberwachungsvorrichtung, einem Operationsfeld, einer Vorrichtung zur Anpassung des Laserstrahls an eine Videoüberwachungsvorrichtung und einem Scansystem, das mit dem optischen Ausgang der Vorrichtung für den CO₂-Laser und dem optischen Eingang der Anpassungsvorrichtung verbunden ist. Darüber hinaus beschreibt die WO 2017/160808 A1 eine Vorrichtung zur Bestimmung der Ausrichtung eines distalen Endes eines medizinischen Instruments, wie z.B. der Elektroden-Gewebe-Ausrichtung eines Hochfrequenz-Ablationskatheters.

EP 3 346 807 B1 beschreibt eine Informationsverarbeitungsvorrichtung mit einem Anwendungspositionsberechnungsabschnitt, der dazu eingerichtet ist, die Anwendungsposition von Plasma auf einem Bearbeitungszielobjekt auf Grundlage von erfassten Bilddaten des Bearbeitungszielobjekts zu berechnen, wenn Plasma von einer Atmosphärendruck-Plasmaerzeugungsvorrichtung auf das Bearbeitungszielobjekt aufgebracht wird.

Davon ausgehend ist es Aufgabe der Erfindung, eine Vorrichtung und ein Verfahren bereitzustellen, die es ermöglichen, dem Anwender eines Instruments, mit dem ein Behandlungsmedium auf eine Gewebeoberfläche frei Hand appliziert werden kann, anzuzeigen, welche Stelle des zu behandelnden Gewebes bereits behandelt wurde.

Diese Aufgabe wird mit der Vorrichtung zur bildgestützten Unterstützung eines Anwenders nach Anspruch 1 gelöst:

Die erfindungsgemäße Vorrichtung weist ein Instrument, eine Bilderfassungseinrichtung, eine Auswertungseinrichtung und eine Wiedergabeeinrichtung auf. Das Instrument ist dazu eingerichtet, ein Behandlungsmedium an veränderbaren Orten in einem zu behandelnden Bereich einer Gewebeoberfläche zu applizieren. Die Bilderfassungseinrichtung ist dazu eingerichtet, den zu behandelnden Bereich zu erfassen. Die Auswertungseinrichtung ist dazu eingerichtet, eine Behandlungsspur des Behandlungsmediums relativ zum zu behandelnden Bereich aus den Daten der Bilderfassungseinrichtung zu bestimmen und anhand der Behandlungsspur eine ortsaufgelöste Dosierung für den zu behandelnden Bereich zu ermitteln. Außerdem ist die Wiedergabeeinrichtung dazu eingerichtet, die Behandlungsspur und/oder die ortsaufgelöste Dosierung für den Anwender in Bezug auf das Gewebe optisch darzustellen.

Hierdurch wird es möglich, dem Anwender optisch anzuzeigen, welche Orte des zu behandelnden Bereichs der Gewebeoberfläche bereits behandelt wurden. Insbesondere wird es möglich, dem Anwender anzuzeigen, welche Dosierung die entsprechenden Stellen erhalten haben. Der Anwender kann hierdurch entscheiden, auf welche Stellen des zu behandelnden Bereichs noch Behandlungsmedium appliziert werden sollte, um eine möglichst gleichmäßige Verteilung des Behandlungsmediums zu erreichen. Die Gefahr einer Überdosierung oder einer Unterdosierung einzelner Stellen des zu behandelnden Bereichs kann so verringert werden.

Dies kann dazu führen, dass die Gleichmäßigkeit, mit der das Behandlungsmedium auf die Gewebeoberfläche appliziert wurde, erhöht werden kann, wobei das Ergebnis der Anwendung hierdurch auswertbar und reproduzierbar ist.

Eine Besonderheit der erfindungsgemäßen Vorrichtung liegt in der Auswertungseinrichtung, mit Hilfe derer die Behandlungsspur des Behandlungsmediums relativ zum behandelnden Bereich bestimmt wird. Es wird dadurch möglich, eine ortsaufgelöste Dosierung für den zu behandelnden Bereich anhand der Behandlungsspur zu ermitteln. Die Behandlungsspur und/oder die ortsaufgelöste Dosierung werden in Bezug auf den zu behandelnden Bereich von der Wiedergabeeinrichtung optisch dargestellt.

Eine ortsaufgelöste Dosierung kann hierbei ein Dosierungswert sein, der einer Stelle im zu behandelten Bereich zugeordnet ist. Dieser Dosierungswert kann binäre Zustände (beispielsweise behandelter oder unbehandelter Zustand) oder auch kontinuierliche Zustände (beispielsweise prozentuale Wertangaben bezogen auf eine vorbestimmte Solldosierung) aufweisen. Die Behandlungsspur kann ein zeitlicher Verlauf der Position und/oder der Größe (wie beispielsweise der Durchmesser oder der Radius) des Auftragungsbereichs sein. Der Auftragungsbereich ist vorzugsweise der Bereich, auf den das Behandlungsmedium auf die Gewebeoberfläche zu einem aktuellen Zeitpunkt durch das Instrument appliziert wird. Dieser Bereich kann beispielsweise elliptisch oder kreisförmig sein. Die Auswertungseinrichtung kann vorzugsweise eine Computer- oder Prozessoreinrichtung sein.

Die Wiedergabeeinrichtung kann eine Lautsprechereinheit aufweisen, mit der die ortsaufgelöste Dosierung akustisch dargestellt werden kann. Beispielsweise kann dazu ein Ton mit veränderlicher Charakteristik (Frequenz (Tonhöhe), Lautstärke oder einer von der Dosierung abhängigen veränderlichen Tonfolge) abgegeben werden. Die Charakteristik kann davon abhängen, ob das Behandlungsmedium schon behandeltes oder noch unbehandeltes Gewebe trifft. Außerdem kann die Veränderung der Toncharakteristik dazu genutzt werden, eine zu lange Verweildauer des Behandlungsmediums auf dem Gewebe anzuzeigen.

Es handelt sich bei dem applizierbaren Behandlungsmedium um ein Plasma, insbesondere ein nieder- oder nichtthermisches Plasma. Das Plasma erzeugt vorzugsweise keinen makroskopisch sichtbaren thermischen Effekt auf der Gewebeoberfläche. Bei der Behandlung mit einem solchen Plasma kann die lokale Temperatur auf der Gewebeoberfläche unterhalb der Denaturierungstemperatur von Proteinen bleiben, also unterhalb einer Temperaturgrenze von 40-70°C, vorzugsweise unter einer Temperatur von 60-70°C. Ein nichtthermisches Plasma ist ein Plasma, das sich nicht im thermischen Gleichgewicht befindet, bei dem sich also beispielsweise die Temperaturen der enthaltenen Teilchen (Ionen, Elektronen oder Neutralteilchen) in einem erheblichen Ausmaß unterscheiden. Bei einem niederthermischen Plasma befindet sich das Plasma ebenfalls nicht im thermischen Gleichgewicht, so dass hierbei auch die Temperaturen der in dem niederthermischen Plasma enthaltenen Teilchen (Ionen, Elektronen oder Neutralteilchen) unterscheiden. Jedoch ist das Ausmaß, mit dem sich die Temperaturen der enthaltenen Teilchen unterscheiden relativ zu einem nichtthermischen Plasma geringer. Bei dem applizierbaren Behandlungsmedium kann es sich außerdem um ein niederenergetisches Argon-Plasma handeln, bei dem an der behandelten Gewebeoberfläche keine sichtbare Veränderung des Gewebes auftritt. Dies zumindest dann nicht, wenn das Plasma nicht länger als eine Maximalzeit von z.B. einigen Sekunden auf die gleiche Stelle des Gewebes trifft.

Es wird bevorzugt, dass die Bilderfassungseinrichtung dazu eingerichtet ist, den zu behandelnden Bereich zumindest teilweise in einer eine Vielzahl von Einzelbildern umfassenden Bildsequenz zu erfassen. Das Behandlungsmedium kann so in den Einzelbildern der Bildsequenz detektiert und verfolgt werden. Die Bildsequenz kann auch ein kontinuierlicher Strom von Einzelbildern sein, wobei ein aktuelles Einzelbild als ein Live-Bild des erfassten Bereichs verstanden wird.

Die Auswertungseinrichtung ist dazu eingerichtet, in der Bildsequenz das Behandlungsmedium und/oder einen Kopf des Instruments zu erkennen und vorzugsweise über mehrere Einzelbilder der Bildsequenz zu verfolgen. Hierdurch kann eine Position des Behandlungsmediums relativ zu dem zu behandelnden Bereich der Gewebeoberfläche bestimmt werden. Für den Fall, dass das Behandlungsmedium im aktuellen Einzelbild von dem Kopf des Instruments verdeckt wird, kann die Position des Kopfes des Instruments dazu dienen, die Position des Behandlungsmediums zu schätzen. Zum Erkennen des Kopfes des Instruments können Bilderkennungsalgorithmen aus dem Bereich des maschinellen Lernens verwendet werden, wie beispielsweise neuronale Netze, *Support-Vector-Machines* oder dergleichen.

Vorzugsweise ist die Auswertungseinrichtung dazu eingerichtet, in einem aktuellen Einzelbild der Bildsequenz eine Position und/oder eine Größe des applizierten Behandlungsmediums zu bestimmen. Dies ermöglicht ein möglichst einfaches und robustes Verfolgen des Behandlungsmediums in der Bildsequenz. Die Position des Behandlungsmediums kann beispielsweise ein Mittelpunkt des in dem Einzelbild dargestellten Auftragungsbereichs sein, in welchem das Behandlungsmedium auf die Gewebeoberfläche trifft. Der Auftragungsbereich kann zum Beispiel eine Ellipse oder ein Kreis sein. Die Größe des applizierten Behandlungsbereichs kann dabei ein Radius oder ein Durchmesser oder eine Fläche des Bereichs sein, in welchem das Behandlungsmedium auf die Gewebeoberfläche trifft.

Insbesondere kann die Auswertungseinrichtung dazu eingerichtet sein, in dem aktuellen Einzelbild der Bildsequenz eine Position, eine Ausrichtung und/oder eine Abmessung des Kopfes des Instruments zu bestimmen. Durch zusätzliche Informationen, wie die Position, die Ausrichtung und/oder die Abmessung des Kopfes, kann die Position des Behandlungsmediums für den Fall, dass das Instrument das Behandlungsmedium verdeckt, bestimmt bzw. geschätzt werden.

In einer vereinfachten Ausführungsform kann auch nur die Position des Kopfes als Maß für die Position des Behandlungsmediums verwendet werden, wodurch auch schwer zu erfassende Behandlungsmedien erfasst und deren Position bestimmt werden können, wie zum Beispiel lichtschwache Behandlungsmedien und/oder Behandlungsmedien, deren Auftrefffläche auf der Gewebeoberfläche unregelmäßig geformt ist. Außerdem kann hierdurch die für die Umsetzung benötigte Rechenleistung reduziert werden.

Bei einer bevorzugten Ausführungsform ist die Auswertungseinrichtung dazu eingerichtet, das Behandlungsmedium in den Daten der Bilderfassungseinrichtung anhand eines Farb- und/oder Formmerkmals zu erkennen. Vorzugsweise ist die Auswertungseinrichtung dazu eingerichtet, das Behandlungsmedium anhand zumindest eines der folgenden Merkmale zu erkennen: einem Farbwechselbereich, einem Helligkeitswertebereich und einem Sättigungswertbereich. Durch die Verwendung eines Farb- und/oder Formmerkmals zur Erkennung des Behandlungsmediums kann das Behandlungsmedium auf möglichst einfache und robuste Weise detektiert werden.

Insbesondere kann die Auswertungseinrichtung dazu eingerichtet sein, den Kopf des Instruments in den Daten der Bilderfassungseinrichtung anhand eines Farb- und/oder Formmerkmals oder anhand eines auf dem Kopf angebrachten Markers zu erkennen. Hierdurch kann der Kopf des Instruments mit besonders einfachen und nicht rechenintensiven Verfahren erkannt werden, woraus die Position des Kopfes in Bezug auf den zu behandelnden Bereich bestimmt werden kann.

Vorzugsweise ist die Auswertungseinrichtung dazu eingerichtet, in dem aktuellen Einzelbild mittels einer Suchroutine in einem Suchbereich das Behandlungsmedium zu erkennen. Der Suchbereich für das Behandlungsmedium kann anhand der Position, der Ausrichtung und/oder der Abmessung des Kopfes verändert werden. Dies ermöglicht es, den Suchbereich für das Behandlungsmedium beispielsweise auf einen kleineren Bereich zu beschränken, der sich an die Spitze des Kopfes des Instruments (unmittelbar) anschließt.

In einer bevorzugten Ausführungsform ist die Auswertungseinrichtung dazu eingerichtet, eine prädizierte Position des Behandlungsmediums im aktuellen Einzelbild anhand einer Position und/oder Geschwindigkeit des Behandlungsmediums aus einem vorherigen Einzelbild zu bestimmen.

Vorzugsweise wird der Suchbereich, in welchem die Suchroutine in dem aktuellen Einzelbild das Behandlungsmedium erkennt, anhand der prädizierten Position verändert. Beispielsweise kann mittels eines *Alpha-Beta-Filters* oder eines *Kalman-Filters* eine Zustandsschätzung für die Position (eine prädizierte Position) des Behandlungsmediums für das aktuelle Einzelbild bestimmt werden. Der Suchbereich der Suchroutine kann um die prädizierte Position herum gelegt werden. Die Größe des Suchbereichs kann hierbei zum Beispiel auf einen vorbestimmten Wert festgelegt sein. Alternativ kann die Größe des Suchbereichs auch beispielsweise anhand der prädizierten Position und/oder der Geschwindigkeit und/oder eines Kovarianzwertes für die prädizierte Position verändert werden. Zum Beispiel kann der Suchbereich bei einem hohen Kovarianzwert verkleinert und umgekehrt bei einem niedrigen Kovarianzwert vergrößert werden. Hierdurch kann das Verfolgen des Behandlungsmediums verbessert werden.

Insbesondere kann der anhand der prädizierten Position des Behandlungsmediums im aktuellen Einzelbild definierte Suchbereich dazu verwendet werden, falsch-positive Fehlzuordnung auszusortieren. Hierbei kann die Suchroutine weiterhin im gesamten aktuellen Einzelbild nach dem Behandlungsmedium suchen. Detektionen, die außerhalb des Suchbereichs liegen, können jedoch als falsch-positive Fehlzuordnung aussortiert werden. Die aussortierten falsch-positive Fehlzuordnungen können beispielsweise direkt verworfen werden. Hierdurch können falsch-positive Fehlzuordnungen reduziert werden.

Zusätzlich oder alternativ, kann die Auswertungseinrichtung dazu eingerichtet sein, dass der zu behandelnde Bereich vor dem Applizieren des Behandlungsmediums von dem Anwender festlegbar ist. Der Anwender kann zum Beispiel vor Beginn des Applizierens mit dem Instrument den zu behandelnden Bereich im Sichtbereich der Bilderfassungseinrichtung nachfahren, ohne Behandlungsmedium zu applizieren. Die Auswertungseinrichtung kann dazu eingerichtet sein, zumindest den Kopf des Instruments zu erkennen und zu verfolgen, um so den gewünschten zu behandelnden Bereich festzulegen. Der so definierte zu behandelnde Bereich kann eine beliebige Form, beliebiger Komplexität aufweisen, da dieser frei Hand von dem Anwender eingegeben werden kann. Alternativ kann die Festlegung des zu behandelnden Bereichs auch durch eine Eingabe des Anwenders beispielsweise auf einem Touchscreen erfolgen. Wenn nun der Anwender Behandlungsmedium auf die Gewebeoberfläche appliziert, können von der Auswertungseinrichtung bestimmte Detektionen des Behandlungsmediums, die außerhalb des zu behandelnden Bereichs liegen, als falsch-positive Fehlzuordnung aussortiert werden. Dies kann es ermöglichen, falsch-positive Fehlzuordnungen zu reduzieren, die beispielsweise durch Lichtreflexionen an metallischen Klemmen außerhalb des zu behandelnden Bereichs eingestreut werden.

Vorzugsweise ist die Auswertungseinrichtung dazu eingerichtet, in der Bilderfassungseinrichtung charakteristische Bildmerkmale des zu behandelnden Bereichs oder in den Bereich der Gewebeoberfläche aufgetragene Referenzmarker zu erkennen und die Behandlungsspur dem zu behandelnden Bereich ortsgetreu zuzuordnen. Zum Erkennen der charakteristischen Bildmerkmale können ebenfalls Bilderkennungsalgorithmen aus dem Bereich des maschinellen Lernens verwendet werden, wie beispielsweise neuronale Netze, insbesondere *Convolutional-Neural-Networks, Support-Vector-Machines* oder dergleichen. Der Referenzmarker kann beispielsweise ein farbiger Punkt oder ein Punkt mit einem besonders gut detektierbaren Muster sein, der im zu behandelnden Bereich angebracht wird.

Bei einer bevorzugten Ausführungsform weist die Wiedergabeeinrichtung eine (*Augmented-Reality-,* AR-) Brille auf, die dazu eingerichtet ist, die Behandlungsspur und/oder die ortsaufgelöste Dosierung des zu behandelnden Bereichs ortsgetreu in das Sichtfeld der Brille zu projizieren.

Die Auswertungseinrichtung ist kommunikativ mit einer Versorgungseinheit des Instruments verbunden, wobei die Auswertungseinrichtung dazu eingerichtet ist, das Applizieren des Behandlungsmediums durch das Instrument in Abhängigkeit von der lokalen Dosierung zu steuern. Hierdurch kann eine Überdosierung einzelner Bereich automatisiert vermieden werden.

Die Auswertungseinrichtung ist kommunikativ mit der Versorgungseinheit des Instruments verbunden, wobei die Auswertungseinrichtung dazu eingerichtet ist, das Ermitteln der ortsaufgelösten Dosierung zusätzlich anhand von Messinformationen der Versorgungseinheit zu bestimmen. Die Messinformationen können von der Versorgungseinheit an die Auswertungseinrichtung übermittelt werden, wodurch das Ermitteln der ortsaufgelösten Dosierung genauer bestimmt werden kann.

In einer konkreten Ausführungsform kann das Instrument Teil einer Behandlungseinrichtung sein, die das Instrument und die Versorgungseinheit umfasst, die das Instrument mit Betriebsmitteln versorgt. Betriebsmittel können beispielsweise Fluide wie Gase oder Flüssigkeiten sein und/oder eine elektrische Spannung, insbesondere eine hochfrequente Wechselspannung. Hierzu kann die Versorgungseinheit einen steuerbaren (Hochfrequenz-, HF-) Generator aufweisen. Der Generator kann die Leistung bereitstellen, welche das Instrument zum Applizieren des Behandlungsmediums benötigt. Ferner kann der Generator eine Steuereinheit aufweisen, die beispielsweise kommunikativ mit der Auswertungseinrichtung verbunden ist. Die Kommunikation zwischen der Steuereinheit und der Auswertungseinrichtung kann bidirektional sein. Beispielsweise können Informationen über die Behandlungsspur und/oder die ortsaufgelöste Dosierung und/oder Messinformationen, wie eine Zünderkennung, ein Strom, eine Spannung, eine Leistung und/oder ein Widerstand, von der Steuereinheit an die Auswertungseinrichtung übertragen werden. Ebenso können andere Bestandteile der Versorgungseinrichtung, beispielsweise eine Gaszufuhr, mit einer Steuereinheit versehen sein und kommunikativ mit der Auswerteeinheit verbunden sein, um beispielsweise Daten zum Gasfluss oder Gasdruck auszutauschen und/oder zu beeinflussen. Die Bestandteile der Versorgungseinheit können dabei separate Geräte sein, die auch ohne das Vorhandensein der jeweils anderen Bestandteile betrieben werden können.

Vorzugsweise ist die Auswertungseinrichtung dazu eingerichtet, den zu behandelnden Bereich in eine Vielzahl von Auflösungselementen aufzuteilen, für die jeweils anhand der Behandlungsspur eine Dosierung bestimmt wird. Ein Auflösungselement kann beispielsweise ein Pixel sein.

Ferner wird die Aufgabe durch das Verfahren zur bildgestützten Unterstützung eines Anwenders nach Anspruch 14 gelöst:
Das erfindungsgemäße Verfahren umfasst ein Erfassen eines zu behandelnden Bereichs, in welchem mit einem Instrument ein Behandlungsmedium an veränderbaren Orten applizierbar ist. Außerdem umfasst das Verfahren das Bestimmen einer Behandlungsspur des Behandlungsmediums relativ zu dem Bereich. Anhand der Behandlungsspur wird eine ortsaufgelöste Dosierung für den zu behandelnden Bereich ermittelt. Darüber hinaus umfasst das Verfahren ein Darstellen der Behandlungsspur und/oder der ortsaufgelösten Dosierung in Bezug auf den zu behandelnden Bereich.

Sämtliche Merkmale und Vorteile, die in Bezug auf die erfindungsgemäße Vorrichtung beschrieben wurden, sind ebenso auf das erfindungsgemäße Verfahren anwendbar.

Weitere Einzelheiten vorteilhafter Weiterbildungen oder Details der Erfindung ergeben sich aus den Zeichnungen der Beschreibung sowie aus den Ansprüchen. Es zeigen:
Figur 1 eine schematische Ansicht eines Ausführungsbeispiels einer Vorrichtung zur bildgestützten Unterstützung eines Anwenders,
Figur 2 eine schematische Ansicht eines weiteren Ausführungsbeispiels einer Vorrichtung zur bildgestützten Unterstützung eines Anwenders,
Figur 3 eine schematische Ansicht eines weiteren Ausführungsbeispiels der erfindungsgemäßen Vorrichtung zur bildgestützten Unterstützung eines Anwenders,
Figuren 4a bis 4c ein Beispiel für einen zu behandelnden Bereich mit Beispielen für dazugehörige Live-Bilder des Bereichs, in welchem die Behandlungsspur dargestellt wird,
Figur 5 ein Beispiel für ein Live-Bild eines zu behandelnden Bereichs,
Figur 6 ein Flussdiagramm für ein Ausführungsbeispiel des erfindungsgemäßen Verfahrens zur bildgestützten Unterstützung eines Anwenders,
Figur 7 ein weiteres Beispiel für ein Live-Bild eines zu behandelnden Bereichs mit prädizierter Position für das Behandlungsmedium.

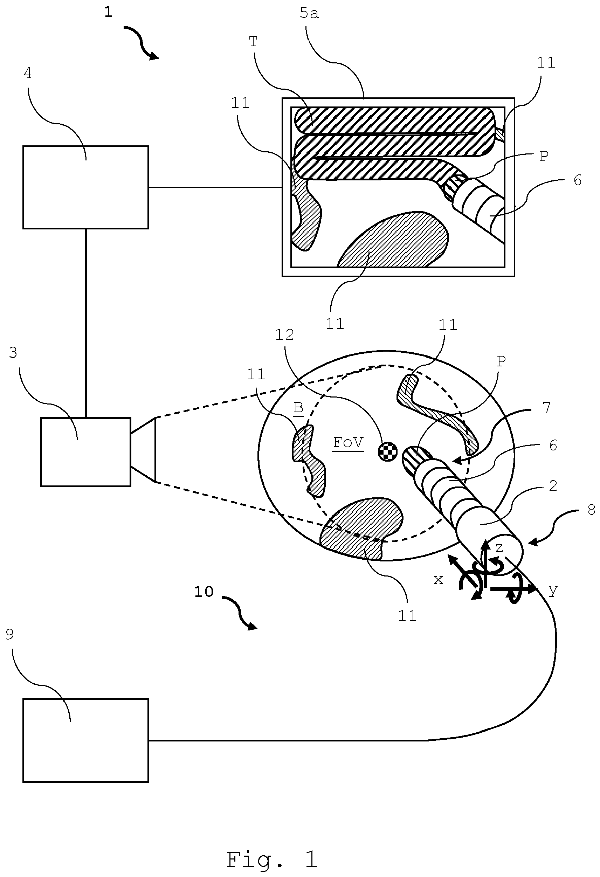

In Figur 1 ist eine Vorrichtung 1 veranschaulicht, die dazu dient, einen Anwender eines (medizinischen) Instruments 2 mittels Bildgebung zu unterstützen.

Die Vorrichtung 1 weist ein Instrument 2 auf, mit dem ein Anwender ein Behandlungsmedium P in einem zu behandelnden Bereich B eines Patienten applizieren kann. Das Instrument 2 kann vom Anwender frei im Raum bewegt werden. Die Bewegung des Instruments kann praktisch beliebigen Pfaden im Raum x, y, z folgen und dabei auch in beliebige Richtungen geschwenkt werden. Durch das Verschieben und/oder Rotieren des Instruments 2 kann der Anwender so den Ort, an welchem das Behandlungsmedium P in einem zu behandelnden Bereich B eine Gewebeoberfläche trifft, variieren.

An einem distalen Ende 7 des Instruments 2 weist das Instrument 2 einen Kopf 6 zur Generierung eines Behandlungsmediums, z.B. eines Plasmas, insbesondere Nichtgleichgewichtsplasmas (Kaltplasmas) auf. Das Plasma tritt aus dem distalen Ende 7 des Instruments 2 aus und trifft auf die Gewebeoberfläche. Das distale Ende 7 des Instruments 2 ist das Ende, welches näher an der Gewebeoberfläche ist, wenn ein Behandlungsmedium appliziert wird. Das proximale Ende 8 des Instruments 2 ist hingegen das Ende, welches weiter entfernt von der Gewebeoberfläche ist, wenn ein Behandlungsmedium appliziert wird. Bei der Gewebeoberfläche kann es sich beispielsweise um Haut, Schleimhaut oder Wunden in der Haut oder Schleimhaut handeln. Dem Instrument 2 wird von einer Versorgungseinheit 9 mit einem HF-Generator elektrische Leistung und ein geeignetes Gas, z.B. Argon zugeführt.

Der zu behandelnde Bereich B wird in diesem Ausführungsbeispiel von einer Bilderfassungseinrichtung 3 teilweise in einem Sichtbereich (Field of View, FoV) der Bilderfassungseinrichtung 3 erfasst. Die Bilderfassungseinrichtung kann beispielsweise eine (Video-)Kamera sein. Vorzugsweise kann die Bilderfassungseinrichtung 3 dazu eingerichtet sein, eine viele Einzelbilder umfassende Bildsequenz zu erzeugen, wobei die Bilderfassungseinrichtung 3 auch ein aktuelles Einzelbild (ein Live-Bild) des zu behandelnden Bereichs B erzeugen kann. Die Bilderfassungseinrichtung 3 kann fest in Bezug auf den zu behandelnden Bereich B oder beweglich (beispielsweise handgehalten) angeordnet sein. Wenn die Bilderfassungseinrichtung beweglich zu dem Bereich B angeordnet ist, kann sich der Sichtbereich FoV in Bezug auf den Bereich B während der Behandlung verschieben.

Die Bilderfassungseinrichtung 3 ist mit einer Auswertungseinrichtung 4 kommunikativ verbunden. Die Auswertungseinrichtung 4 ist dazu eingerichtet, Daten der Bilderfassungseinrichtung 3 zu empfangen und auszuwerten. In der Auswertungseinrichtung 4 kann eine Behandlungsspur T des Behandlungsmediums P in Bezug auf den zu behandelnden Bereich B aus den Daten der Bilderfassungseinrichtung 3 bestimmt werden. Anhand der Behandlungsspur T kann nun in der Auswertungseinrichtung 4 eine ortsaufgelöste Dosierung D für den zu behandelnden Bereich ermittelt werden. Wenn die Bilderfassungseinrichtung 3 beweglich zu dem Bereich B angeordnet ist, können in der Auswertungseinrichtung 4 beispielsweise charakteristische Merkmale 11 des zu behandelnden Bereichs B in den Einzelbildern der Bildsequenz erkannt und die Bewegungen der Bilderfassungseinrichtung 3 kompensiert werden.

Die Auswertungseinrichtung 4 ist außerdem kommunikativ mit einer Wiedergabeeinrichtung 5 verbunden. In diesem Ausführungsbeispiel ist die Wiedergabeeinrichtung 5 als eine Anzeigeeinrichtung 5a, beispielsweise in Form eines Bildschirms, ausgestaltet. In dem vorliegenden Beispiel ist die Wiedergabeeinrichtung 5 dazu eingerichtet, die Behandlungsspur T für den Anwender optisch virtuell auf dem Bild des Gewebes darzustellen. Die Wiedergabeeinrichtung 5 kann die Transparenz oder Farbintensität der eingeblendeten Behandlungsspur T entsprechend der lokalen Dosierung D verändern. Damit wird die an sich spurlose Einwirkung des Mediums auf das Gewebe als Spur sichtbar gemacht.

In Figur 1 wird in einem Live-Bild des zu behandelnden Bereichs die Behandlungsspur ortsgetreu eingeblendet. In dem Live-Bild in Figur 1 sind die charakteristischen Merkmale 11 ebenfalls abgebildet. Die Auswertungseinrichtung 4 ist darüber hinaus dazu eingerichtet, die charakteristischen Merkmale 11 in den Einzelbildern der Bildsequenz zu erkennen und die Behandlungsspur T relativ zu der Position der charakteristischen Merkmale für ein aktuelles Einzelbild (Live-Bild) der Bildsequenz zu berechnen und in dieses einzublenden.

Zusätzlich oder alternativ kann ein Referenzmarker 12 in dem zu behandelnden Bereich B auf der Gewebeoberfläche aufgeklebt werden. Der Referenzmarker 12 kann beispielsweise ein optisches Muster aufweisen, das besonders einfach von der Auswertungseinrichtung 4 erkannt werden kann. In einer bevorzugten Ausführungsform sind zwei oder mehr Referenzmarker auf der Gewebeoberfläche aufgeklebt, um eine eindeutige Bestimmung der räumlichen Orientierung jedes Einzelbildes zu ermöglichen.

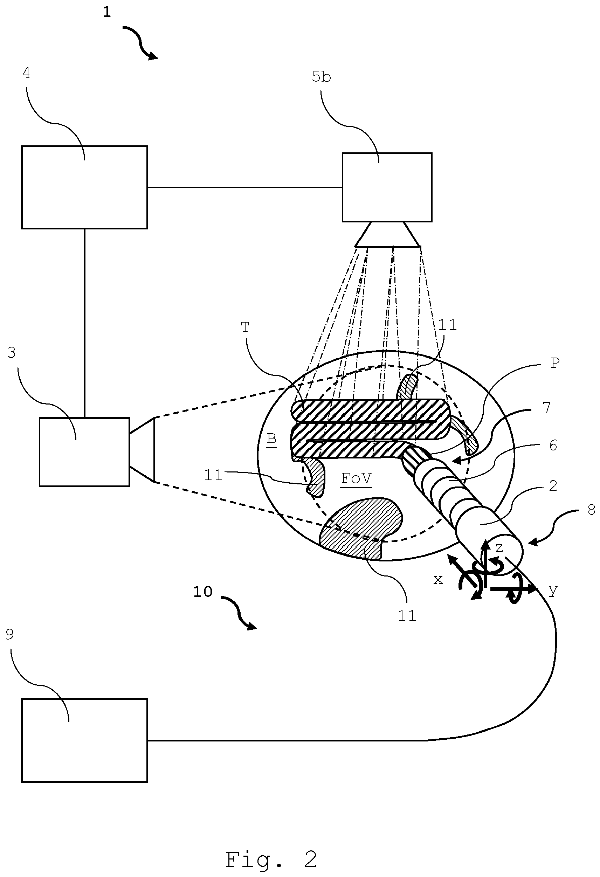

In Figur 2 ist ein weiteres Ausführungsbeispiel der erfindungsgemäßen Vorrichtung veranschaulicht. Anstelle der Anzeigeeinrichtung 5a wird in diesem Beispiel die Behandlungsspur T und die ortsaufgelöste Dosierung durch eine Projektionseinrichtung 5b direkt in den zu behandelnden Bereich B projiziert. Die Projektionseinrichtung 5b kann beispielsweise eine Laser- oder Leuchtdiode als Lichtquelle enthalten sowie weitere optische Komponenten wie Linsen und Spiegel, um die Behandlungsspur T entsprechend darzustellen.

Alternativ oder zusätzlich kann die Wiedergabeeinrichtung 5 auch eine Augmented-Reality (AR-) Brille 5c aufweisen, die auch mit der Auswertungseinrichtung 4 kommunikativ verbunden sein kann, beispielsweise drahtgebunden oder drahtlos. Die AR-Brille 5c ist dazu eingerichtet, die ortsaufgelöste Dosierung des zu behandelnden Bereichs ortsgetreu in das Sichtfeld der Brille zu projizieren.

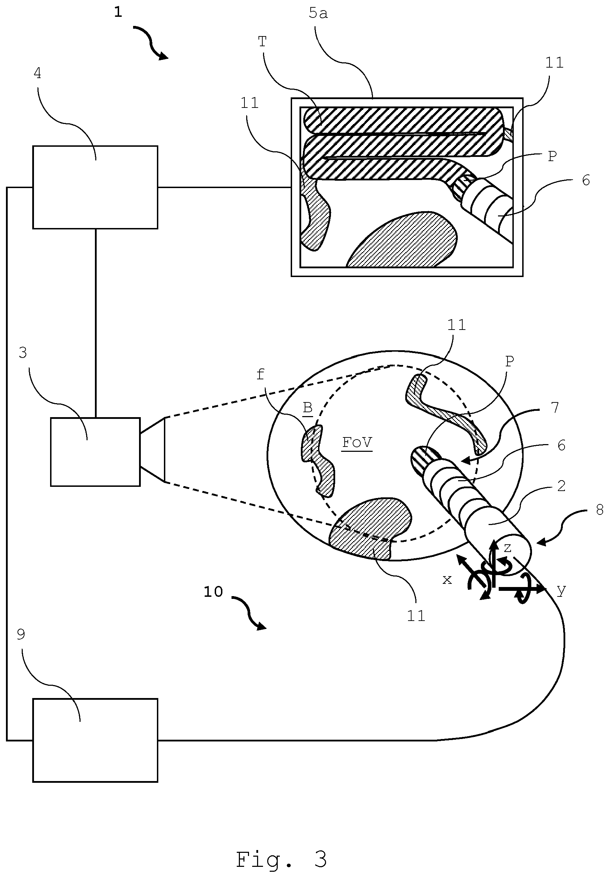

In Figur 3 ist ein weiteres Ausführungsbeispiel der erfindungsgemäßen Vorrichtung 1 veranschaulicht. Das Beispiel aus Figur 3 entspricht im Wesentlichen dem Ausführungsbeispiel aus Figur 1, mit dem Unterschied, dass die kommunikative Verbindung zwischen der Auswertungseinrichtung 4 und der Versorgungseinheit 9, die unter anderem einen HF-Generator sowie eine Gaszufuhr enthält und das Instrument 2 speist, dargestellt ist.

Durch diese kommunikative Verbindung kann die Auswerteeinrichtung 4 auf Mess- und Steuerdaten der Versorgungseinheit 9 zugreifen und diese zur Berechnung der Dosierung nutzen. Die Mess- oder Steuerdaten können beispielsweise für den HF-Generator die Zünderkennung, die Leistung, die Ausgangsleistung, der Strom, die Spannung oder der Widerstand sein oder im Falle der Gaszufuhreinheit der Gasstrom oder der Gasdruck sein.

In einem möglichen Ausführungsbeispiel können in der Auswertungseinrichtung 4 auch Werte für eine ideale Dosierung und/oder Grenzwerte für eine minimale und/oder maximale Dosierung des Gewebes hinterlegt sein. Diese können entweder fest vorgegeben sein oder durch eine geeignete Eingabeeinrichtung durch den Nutzer individuell, beispielsweise abgestimmt auf den Patienten oder den durchzuführenden Eingriff, festgelegt werden.

Die Auswertungseinrichtung 4 kann in diesem Ausführungsbeispiel dazu eingerichtet sein, in Abhängigkeit von der ermittelten lokalen Dosierung für den Bereich, in welchem sich der Kopf 6 des Instruments 2 aktuell befindet, das Applizieren des Behandlungsmediums P zu verändern. So kann beispielsweise die Applikation des Behandlungsmediums durch das Instrument gedrosselt werden, wenn der aktuell behandelte Bereich bereits nahe der hinterlegten gewünschten oder maximalen Dosierung liegt, das heißt, wenn beispielsweise bereits 70 % oder 80 % oder 90 % der gewünschten oder maximalen Dosierung erreicht sind. Die Applikation des Behandlungsmediums kann auch komplett abgeschaltet werden, wenn der zu behandelnde Bereich bereits mit der gewünschten oder maximalen Dosierung behandelt wurde. Wenn der Kopf des Instruments anschließend über einen Gewebebereich bewegt wird, dessen gewünschte oder maximale Dosierung noch nicht erreicht ist, wird die Applikation des Behandlungsmediums wieder erhöht bzw. zugelassen. Dies kann beispielsweise dadurch erreicht werden, dass die Auswertungseinrichtung 4 einer Steuereinheit des HF-Generators in der Versorgungseinheit 9 Informationen über die Behandlungsspur T oder die lokale Dosierung D zur Verfügung stellt. Auch ist es möglich, dass die Auswertungseinrichtung 4 der Steuereinheit ein Signal zum Drosseln oder zur Erhöhung der Leistung oder auch zum Abschalten bzw. zum Einschalten der Leistung schickt, die dem Instrument 2 durch den HF-Generator der Versorgungseinheit 9 bereitgestellt wird.

Die ortsaufgelöste Dosierung kann aus der Verweildauer des Behandlungsmediums P auf den einzelnen Stellen im zu behandelnden Bereich B und den eingestellten Behandlungsparametern berechnet werden, wie beispielsweise einem Behandlungsmodus und/oder einer Behandlungsleistung. In einem vereinfachten Ausführungsbeispiel kann auch nur die Verweildauer für die Berechnung der ortsaufgelösten Dosierung herangezogen werden, zum Beispiel, wenn davon ausgegangen werden kann, dass für den Eingriff ein vorbestimmter Behandlungsmodus und/oder eine vorbestimmte Behandlungsleistung verwendet wird.

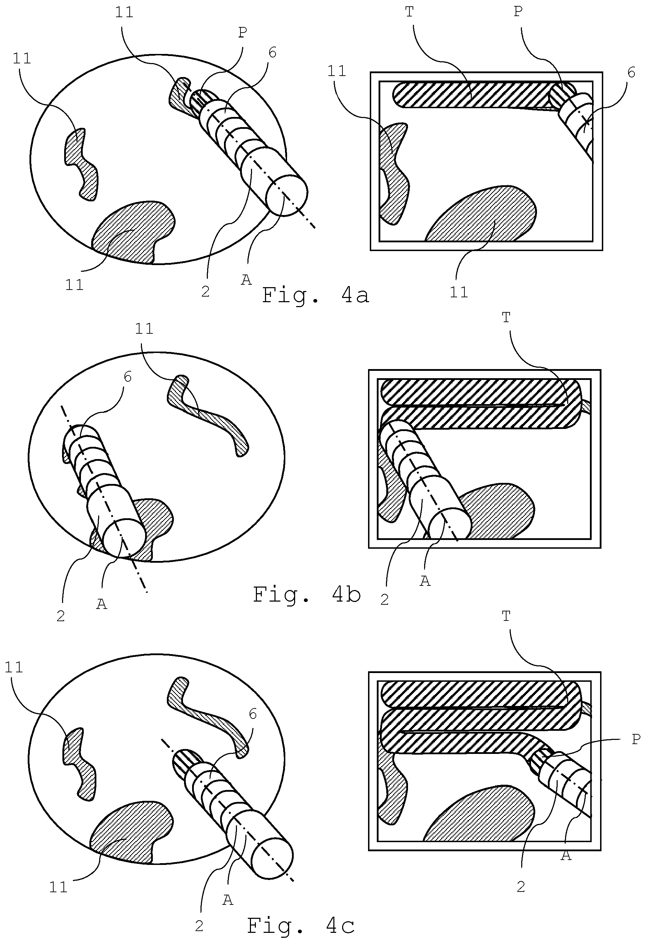

In den Figuren 4a bis 4c ist ein Beispiel für einen zu behandelnden Bereich B mit einem dazu gehörigen Live-Bild veranschaulicht.

Auf der linken Seite ist in den Figuren 4a bis 4c jeweils der zu behandelnde Bereich B gezeigt, wobei mit dem Instrument 2 das Plasma P auf die Gewebeoberfläche G appliziert wird. Auf der rechten Seite der Figuren 4a bis 4c ist jeweils ein dazugehöriges Live-Bild abgebildet.

In dem Live-Bild in Figur 4a wurde von der Auswertungseinrichtung 4 die aktuelle Position des Plasmas P in Bezug auf den zu behandelnden Bereich B bestimmt. Darüber hinaus ist in dem Live-Bild die Behandlungsspur T abgebildet, also der zeitliche Verlauf der Position des Plasmas P in dem zu behandelnden Bereich B.

Alternativ oder zusätzlich kann anstelle der Behandlungsspur T auch die Dosierung D in das Live-Bild eingeblendet werden. Dies wird beispielsweise dadurch erreicht, dass die Farbe oder die Transparenz der eingeblendeten Behandlungsspur T im Live-Bild in Abhängigkeit von der lokalen Dosierung D variiert wird.

In Figur 4b ist das Beispiel des zu behandelnden Bereichs B aus Figur 4a abgebildet. Im Vergleich zu Figur 4a wurde das Instrument 2 weiter verschoben, so dass der Ort, an dem das Instrument 2 das Behandlungsmedium P appliziert, verändert wurde. In Figur 4b ist das Behandlungsmedium P von dem Kopf 6 des Instruments 2 verdeckt. Für die Auswertungseinrichtung 4 ist es hierdurch nicht möglich, direkt das Behandlungsmedium P in dem aktuellen Einzelbild der Bildsequenz zu finden. In diesem Fall ermittelt die Auswerteeinrichtung 4 die Position und die Ausrichtung des Kopfes 6 des Instruments 2 relativ in Bezug auf den zu behandelnden Bereich B.

Hierzu bestimmt die Auswerteeinrichtung 4 beispielsweise eine Hauptachse A des Kopfes 6 des Instruments 2 und anhand der Hauptachse A wird eine distale Richtung des Kopfes 6 ermittelt. Anhand der Position und der Ausrichtung des Kopfes 6 bestimmt die Auswerteeinrichtung 4 die Position des verdeckten Behandlungsmediums P. Als aktuelle Position des Behandlungsmediums P auf der Gewebeoberfläche kann die Auswerteeinrichtung 4 in einem Ausführungsbeispiel das distale Ende 7 des Kopfes 6 des Instruments 2 verwenden.

In einem weiteren Ausführungsbeispiel kann die Auswertungseinrichtung durch eine Extrapolation der Position des Kopfes 6 entlang der durch die Hauptachse A des Instrumentenkopfes 6 bestimmten Ausrichtung des Instruments 2 bestimmt werden. Hierdurch kann auch im Falle von einer Verdeckung des Behandlungsmediums P im aktuellen Einzelbild ein genaues Bestimmen des Behandlungsmediums erreicht werden.

In Figur 4c ist der zu behandelnde Bereich B aus Figuren 4a und 4b abgebildet, wobei das Instrument 2 von dem Anwender verschoben wurde, so dass der aktuelle Ort an dem das Instrument das Behandlungsmedium P appliziert, verändert wurde.

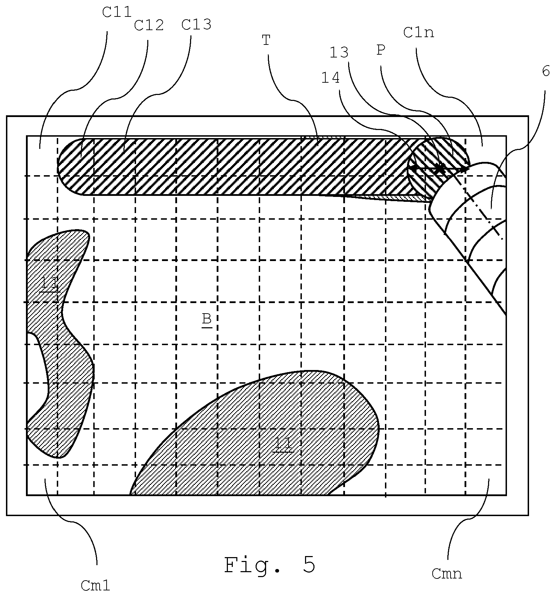

In Figur 5 ist ein vergrößerter Ausschnitt eines Live-Bildes schematisch veranschaulicht. Der in dem Live-Bild abgebildete zu behandelnde Bereich B ist in eine Vielzahl von Auflösungselementen C11 bis Cmn aufgeteilt. Für jedes Auflösungselement wird eine Dosierung D bestimmt, die anhand der Behandlungsspur T berechnet wird. In Figur 5 ist beispielsweise in dem Auflösungselement C11 kein Behandlungsmedium P appliziert worden, da die Behandlungsspur T nicht durch das Auflösungselement C11 läuft. Die Dosierung D11 des Auflösungselement C11 beträgt somit den Wert 0. Die Behandlungsspur T durchläuft in diesem Beispiel das Auflösungselement C12 teilweise. Für das Auflösungselement C12 kann die Auswerteeinrichtung 4 somit ein Maß für die Dosierung D12 zum einen aus der Zeitdauer bestimmen, für die das Behandlungsmedium P innerhalb des Auflösungselements C12 liegt. Zum anderen kann die Auswerteeinrichtung 4 in das Maß für die Dosierung D12 auch den Anteil der Fläche der Auflösungszelle einbeziehen, auf die das Behandlungsmedium P appliziert wurde. Dies kann insbesondere für die Genauigkeit der Dosierung D12 in einem Randbereich des Behandlungsmediums P von Vorteil sein. Wenn zum Beispiel im aktuellen Einzelbild die Position des Behandlungsmediums P als Mittelpunkt des detektierten Behandlungsmediums P bestimmt wird und eine Umrandung des Behandlungsmediums P im aktuellen Einzelbild um den bestimmten Mittelpunkt herum gelegt wird, kann die Umrandung des Behandlungsmediums P zwischen mehreren Auflösungszellen verlaufen, so dass insbesondere im Randbereich bei manchen Auflösungszellen ein Flächenanteil von dem Behandlungsmedium P behandelt wird. Vorteilhaft ist eine Kombination der beiden Wege, in der sowohl die zeitliche Verweildauer des Behandlungsmediums P im Auflösungselement als auch der behandelte Flächenanteil miteinbezogen werden. In Figur 5 kann die Auswerteeinrichtung 4 die aktuelle Position 13 des Behandlungsmediums P beispielsweise als Mittelpunkt des zumindest im Wesentlichen kreisförmigen Bereichs bestimmen, in welchem das Behandlungsmedium P aktuell appliziert wird. Auch kann beispielsweise die Auswerteeinrichtung 4 den Durchmesser 14 dieses Bereichs als Größe des aktuell applizierten Behandlungsmediums bestimmen.

Ebenfalls kann die Auswerteeinrichtung 4 Messwerte der Steuerung des Instruments 2, beispielsweise eine Zünderkennung, einen Strom, eine Spannung, eine Leistung oder einen Widerstand dazu verwenden, die Genauigkeit der aus den Positionsverlauf des Behandlungsmediums P (der Behandlungsspur T) und der daraus ermittelten Dosierung zu verbessern. Eine Verknüpfung zwischen den Messwerten und der Behandlungsspur kann über die Zeitinformationen erreicht werden.

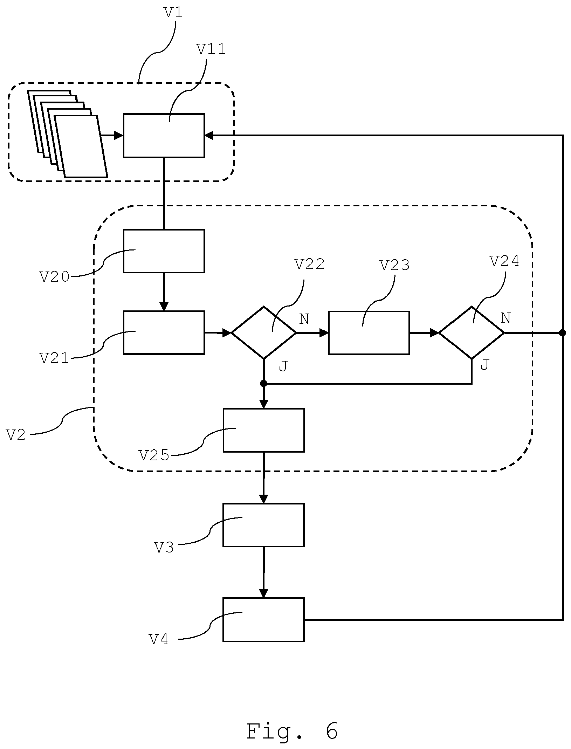

In Figur 6 ist ein Flussdiagramm für ein Beispiel des von der Auswerteeinrichtung 4 und den übrigen Komponenten der Vorrichtung 2 ausgeübten erfindungsgemäßen Verfahrens zur bildgestützten Unterstützung eines Anwenders abgebildet.

Im Verfahrensschritt V1 wird der zu behandelnde Bereich B erfasst, in welchem mit einem Instrument 2 Behandlungsmedium P an manuell veränderbaren Orten appliziert werden kann. Beispielsweise kann der Bereich B in einer eine Vielzahl von Einzelbildern umfassenden Bildersequenz erfasst werden. Für die weiteren Verarbeitungsschritte der Verfolgungsschleife (*Tracking-Loop*) wird nun beispielsweise ein aktuelles Einzelbild verwendet (Verfahrensschritt V11).

In Verfahrensschritt V2 kann nun eine Behandlungsspur T des Behandlungsmediums P relativ zu dem Bereich B bestimmt. Hierfür kann zunächst in einem Verfahrensschritt V20 ermittelt werden, ob sich der Sichtbereich FoV der Bilderfassungseinrichtung 3 in Bezug auf den Bereich B im aktuellen Einzelbild verschoben hat. Eine Verschiebung des Sichtbereichs FoV kann zum Beispiel aufgrund einer Bewegung, wie einer Translation und/oder Rotation, der Bilderfassungseinrichtung 3 relativ zur Gewebeoberfläche oder umgekehrt vorkommen. Die Verschiebung kann durch einen Vergleich der Position und Lage von charakteristischen Merkmalen 11 im aktuellen Einzelbild mit der Position und Lage der charakteristischen Merkmale 11 in einem vorherigen Einzelbild bestimmt werden. Auf diese Weise lassen sich auftretende Verschiebungen quantitativ erfassen und kompensieren. Anschließend kann in dem aktuellen Einzelbild nach dem Behandlungsmedium P gesucht und die Position 13 und/oder die Größe 14 des aktuell aufgetragenen Behandlungsmediums P bestimmt werden (Verfahrensschritt V21). Falls das Behandlungsmedium P in dem aktuellen Einzelbild nicht gefunden werden kann (Abfrage in Verfahrensschritt V22), kann nach dem Kopf 6 des Instruments 2 gesucht und die Position, die Ausrichtung und/oder die Größe des Kopfes 6 bestimmt werden (Verfahrensschritt V23).

Falls das Behandlungsmedium P in dem aktuellen Einzelbild gefunden wurde, also eine aktuelle Position 13 und/oder Größe 14 des Behandlungsmediums P für das aktuelle Einzelbild bestimmt wurde, kann in dem Verfahrensschritt V25 die Behandlungsspur T bestimmt bzw. aktualisiert werden. Hierfür kann beispielsweise die ermittelte, aktuelle Position 13 sowie die Größe des Behandlungsmediums P für jedes entsprechende Einzelbild gespeichert werden, so dass sich aus der Gesamtheit der Positionen 13 und Größen 14 der Einzelbilder eine Behandlungsspur T in der Bildsequenz ergibt. Die ortsaufgelöste Dosierung D kann nun durch eine Auswertung des örtlichen Verlaufs und vorzugsweise des zeitlichen Verlaufs der Position und Größe des Behandlungsmediums (der Bewegungsspur) ermittelt werden (weiterhin Verfahrensschritts V25). Falls die Position des Behandlungsmediums P nicht direkt in einem aktuellen Einzelbild gefunden, dafür aber die Position des Kopfes 6 des Instruments 2 bestimmt wurde (Abfrage in Verfahrensschritt V24), kann die Position des Behandlungsmedium P anhand der Position, der Ausrichtung und/oder der Größe des Kopfes 6 des Instruments 2 bestimmt (geschätzt) werden. Falls auch keine Position des Kopfes 6 des Instruments 2 gefunden wird, wird das Verfahren mit Verfahrensschritt V11 fortgesetzt, in welchem nun ein neues, aktuelles Einzelbild als Datengrundlage für die Unterschritte V21 bis V25 des Verfahrensschritts V2 verwendet wird.

Ebenfalls möglich ist, dass die Schritte V21/V22 und V23/V24 in der Reihenfolge vertauscht werden. So können zunächst die Position, die Ausrichtung und/oder die Größe des Kopfes 6 bestimmt werden. Anhand dieser Parameter kann nun ein Suchgebiet, in welchem die Suchroutine in dem aktuellen Einzelbild nach dem Behandlungsmedium P sucht, verändert werden. Vorzugsweise beschränkt sich nun der Suchbereich auf einen sich an das distale Ende 7 des Kopfes 6 angrenzenden Bereich. Es ist hierdurch nicht mehr notwendig, das gesamte aktuelle Einzelbild nach dem Behandlungsmedium P abzusuchen.

Darüber hinaus ist es möglich, dass die Verfahrensschritte V21 und V23 parallel ausgeführt werden.

In einem vereinfachten Ausführungsbeispiel kann anstelle der Position des Behandlungsmediums P (Unterschritte V21 und V22) auch nur die Position des Kopfes 6 (Unterschritt V23) als Maß für die Position des Behandlungsmediums P verwendet werden. In diesem Beispiel werden also nur die Unterschritte V23 bis V25 des Verfahrensschritts V2 durchgeführt.

Die Behandlungsspur T wird vorzugsweise in Verfahrensschritt V25 entsprechend der geschätzten Position des Behandlungsmediums P aktualisiert werden.

Anhand der Behandlungsspur T kann in dem Verfahrensschritt V3 eine ortsaufgelöste Dosierung von dem zu behandelnden Bereich B ermittelt werden.

Im Verfahrensschritt V4 kann nun die Behandlungsspur T und/oder die ortsaufgelöste Dosierung D in Bezug auf den zu behandelnden Bereich B dargestellt werden, indem die Behandlungsspur in das Live-Bild eingeblendet wird.

Nach Verfahrensschritt V4 folgt erneut Verfahrensschritt V11, in welchem ein neues, aktuelles Einzelbild als Datengrundlage für die Verfahrensschritte der Verfolgungsschleife verwendet wird.

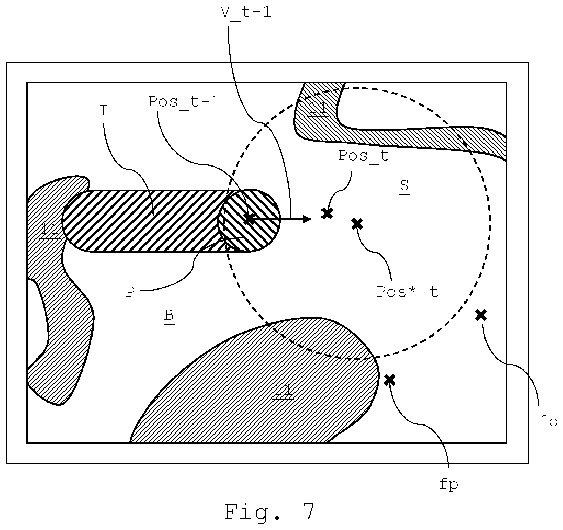

In Figur 7 ist ein vergrößerter Ausschnitt eines Live-Bildes schematisch veranschaulicht. In diesem Ausführungsbeispiel ist die Auswertungseinrichtung dazu eingerichtet, eine prädizierte Position Pos*_t des Behandlungsmediums P für das aktuelle Einzelbild zu bestimmen. Die prädizierte Position Pos*_t kann anhand der vorherigen Position Pos_t-1 und/oder der vorherigen Geschwindigkeit V_t-1 des Behandlungsmediums P bestimmt werden. In diesem Beispiel wird der Suchbereich S, in welchem die Suchroutine in dem aktuellen Einzelbild das Behandlungsmedium erkennt, um die prädizierte Position Pos*_t herum festgelegt. Die im aktuellen Einzelbild erfasste Position Pos_t des Behandlungsmediums P liegt innerhalb des Suchbereichs S.

Alternativ kann auch der festgelegte Suchbereich S dazu verwendet werden, Detektionen fp, bei denen das Behandlungsmedium P beispielsweise aufgrund von Lichtreflexionen außerhalb des Suchbereichs S detektiert wird, als falsch-positive Fehlzuordnung auszusortieren.

Es besteht außerdem die Möglichkeit, dass die Größe des Suchbereichs S in Abhängigkeit der Geschwindigkeit V_t-1 angepasst wird. Ist die Geschwindigkeit V_t-1 hoch, wird der Suchbereich S größer gewählt. Ist die Geschwindigkeit V_t-1 hingegen gering, kann auch der Suchbereich S kleiner gefasst werden.

Die insoweit beschriebene Vorrichtung 1 zur bildgestützten Unterstützung des Anwenders arbeitet wie folgt:

Zur Durchführung einer Behandlung kann der Anwender mit dem Instrument 2, wie beispielsweise einem Plasma-Applikator, frei Hand, in gewünschter Dosierung Plasma in einem zu behandelnden Bereich auf eine Gewebeoberfläche applizieren. Der Anwender kann während der Behandlung beispielsweise auf eine Anzeigeeinrichtung 5a schauen, auf der der zu behandelnde Bereich der Gewebeoberfläche abgebildet ist und/oder eine AR-Brille 5c tragen. Während der Behandlung kann außerdem die Behandlungsspur T und/oder die Dosierung D durch eine Projektionseinrichtung 5b für den Anwender sichtbar in den zu behandelnde Bereich auf die Gewebeoberfläche projiziert werden.

Beim Durchführen der Behandlung wird nun dem Anwender angezeigt, welche Orte des zu behandelnden Bereichs bereits mit dem Plasma behandelt wurden. Vorzugsweise wird dem Anwender außerdem angezeigt, welche Dosierung die einzelnen Orte im zu behandelnden Bereich erhalten haben. Dem Anwender wird es so ermöglicht, an unbehandelten Stellen oder Stellen mit einer zu niedrigen Dosierung Plasma zu applizieren.

Beispielsweise wird im Falle einer Behandlung einer *cervikalen intraepithelialen Neoplasie* (CIN) ein zu behandelnder Bereich der Schleimhautoberfläche mit einem Video-Kolposkop betrachtet. Die Auswertungseinrichtung kann in diesem Fall mit dem üblicherweise bereits vorhandenen Video-Kolposkop verbunden werden, so dass die Auswertungseinrichtung die (Video-) Daten des Video-Kolposkops auswerten kann. Gleichermaßen kann bei anderen Eingriffen beispielsweise die Kamera eines Endoskops oder Laparoskops verwendet werden.

Die Behandlung einer CIN kann beispielsweise dadurch erfolgen, dass ein nichtthermisches Plasma auf die Schleimhautoberfläche appliziert wird. Die Daten des Video-Kolposkops können nun von der Auswertungseinrichtung verwendet werden, um den zeitlichen Verlauf der Position des Plasmas zu bestimmen (Behandlungsspur) und daraus die ortsaufgelöste Dosierung zu ermitteln. Die Behandlungsspur oder ortsaufgelöste Dosierung kann entsprechend auf dem Bildschirm des Video-Kolposkops und/oder auf weiteren Bildschirmen angezeigt werden.

Die erfindungsgemäße Vorrichtung 1 dient zur bildgestützten Unterstützung eines Anwenders eines Instruments 2. Das Instrument 2 ist dazu eingerichtet, ein Behandlungsmedium an veränderbaren Orten in einem zu behandelnden Bereich einer Gewebeoberfläche zu applizieren. Außerdem weist die Vorrichtung eine Bilderfassungseinrichtung auf, die dazu eingerichtet ist, den zu behandelnden Bereich zu erfassen. Die Vorrichtung 1 weist eine Auswertungseinrichtung 4 auf, die dazu eingerichtet ist, eine Behandlungsspur des Behandlungsmediums relativ zu dem behandelnden Bereich aus den Daten der Bilderfassungseinrichtung zu bestimmen und eine ortsaufgelöste Dosierung für den zu behandelnden Bereich anhand der Behandlungsspur zu ermitteln. Die Vorrichtung 1 weist auch eine Wiedergabeeinrichtung 5 auf, die dazu eingerichtet ist, die Behandlungsspur und/oder die ortsaufgelöste Dosierung für den Anwender optisch in Bezug auf das Gewebe darzustellen. Durch die erfindungsgemäße Vorrichtung 1 wird es ermöglicht, dem Anwender optisch anzuzeigen, welche Orte des zu behandelnden Bereichs der Gewebeoberfläche bereits behandelt wurden und/oder mit welcher Dosierung die entsprechend behandelten Orte behandelt wurden.

### Bezugszeichen:

- 1: Vorrichtung zur bildgebenden Unterstützung eines Anwenders
- 2: Instrument
- 3: Bilderfassungseinrichtung (Kamera)
- 4: Auswertungseinrichtung
- 5: Wiedergabeeinrichtung
- 5a: Bildschirm
- 5b: Projektionseinrichtung
- 5c: (AR-)Brille
- 6: Kopf des Instruments
- 7: Distales Ende des Instruments
- 8: Proximales Ende des Instruments
- 9: Versorgungseinheit (HF-Generator, Gaszufuhr)
- 10: Behandlungseinrichtung
- 11: charakteristische Merkmale des Bereichs
- 12: Referenzmarker
- 13: Position des Behandlungsmediums
- 14: Größe (Durchmesser) des Behandlungsmediums
- A: Hauptachse des Instruments
- B: Bereich, der zu behandeln ist
- C11, ..., Cmn: Auflösungselemente
- D: Dosierung
- fp: falsch-positive Fehlzuordnung
- FoV: Sichtbereich
- P: Behandlungsmedium (nichtthermisches Plasma)
- Pos_t: Position des Behandlungsmediums in einem aktuellen Einzelbild
- Pos_t-1: Position des Behandlungsmediums in einem vorherigen Einzelbild
- V_t-1: Geschwindigkeit des Behandlungsmediums in einem vorherigen Einzelbild
- Pos*_t: prädizierte Position des Behandlungsmediums in einem aktuellen Einzelbild
- T: Behandlungsspur
- S: Suchbereich
- V1: Verfahrensschritt des Erfassens des zu behandelnden Bereichs
- V2: Verfahrensschritt des Bestimmens der Behandlungsspur
- V3: Verfahrensschritt des Ermittelns der ortsaufgelösten Dosierung
- V4: Verfahrensschritt des Darstellens der Behandlungsspur und/oder der Dosierung
- V11: Verfahrensunterschritt des Heranziehens eines aktuellen Einzelbildes
- V20: Verfahrensunterschritt des Feststellens der möglichen Veränderung des Sichtbereich FoV in Bezug auf den Bereich B
- V21: Verfahrensunterschritt des Suchens nach dem Behandlungsmedium (Bestimmen von Position und/oder Größe des Behandlungsmediums)
- V22: Abfrageschritt, ob das Behandlungsmedium gefunden wurde
- V23: Verfahrensunterschritt des Suchens nach dem Kopf des Instruments (Bestimmen von Position, Ausrichtung und/oder Größe des Kopfes)
- V24: Abfrageschritt, ob der Kopf des Instruments gefunden wurde
- V25: Verfahrensunterschritt des Bestimmens bzw. Aktualisierens der Behandlungsspur
- x: X-Achse des Instruments
- y: Y-Achse des Instruments
- z: Z-Achse des Instruments

## Patentansprüche

1. Vorrichtung (1) zur bildgestützten Unterstützung eines Anwenders, aufweisend:
- ein Instrument (2), das dazu eingerichtet ist, ein Behandlungsmedium (P) an veränderbaren Orten in einem zu behandelnden Bereich (B) zu applizieren, wobei das applizierbare Behandlungsmedium (P) ein Plasma ist;
- eine Bilderfassungseinrichtung (3), die dazu eingerichtet ist, den zu behandelnden Bereich (B) zu erfassen;
- eine Auswertungseinrichtung (4), die dazu eingerichtet ist,
eine Behandlungsspur (T) des Behandlungsmediums (P) relativ zum behandelnden Bereich (B) aus den Daten der Bilderfassungseinrichtung (3) zu bestimmen, und
eine ortsaufgelöste Dosierung (D) für den zu behandelnden Bereich (B) anhand der Behandlungsspur (T) zu ermitteln; sowie
- eine Wiedergabeeinrichtung (5, 5a, 5b, 5c), die dazu eingerichtet ist, die Behandlungsspur (T) und/oder die ortsaufgelöste Dosierung (D) in Bezug auf den zu behandelnden Bereich (B) für den Anwender optisch darzustellen,
wobei die Auswertungseinrichtung (4) dazu eingerichtet ist, das Behandlungsmedium (P) und/oder einen Kopf (6) des Instruments (2) in einer Bildsequenz des zu behandelnden Bereichs (B) zu erkennen, **dadurch gekennzeichnet,**
**dass** die Auswertungseinrichtung (4) kommunikativ mit einer Versorgungseinheit (9) des Instruments (2) verbunden ist,
wobei die Auswertungseinrichtung (4) dazu eingerichtet ist, das Ermitteln der ortsaufgelösten Dosierung (D) zusätzlich anhand von Messparametern zu bestimmen, die von der Versorgungseinheit (9) an die Auswertungseinrichtung (4) übermittelt werden.

2. Vorrichtung (1) nach Anspruch 1, **dadurch gekennzeichnet, dass** das applizierbare Behandlungsmedium (P) ein nieder- oder nichtthermisches Plasma ist.

3. Vorrichtung (1) nach Anspruch 1 oder 2, **dadurch gekennzeichnet, dass** die Bilderfassungseinrichtung (3) dazu eingerichtet ist, den zu behandelnden Bereich (B) in der eine Vielzahl von Einzelbildern umfassenden Bildsequenz zu erfassen.

4. Vorrichtung (1) nach einem der vorhergehenden Ansprüche, **dadurch gekennzeichnet, dass** die Auswertungseinrichtung (4) dazu eingerichtet ist, das Behandlungsmedium (P) und/oder den Kopf (6) des Instruments (2) über mehrere Einzelbilder der Bildsequenz zu verfolgen.

5. Vorrichtung (1) nach Anspruch 3 oder 4, **dadurch gekennzeichnet, dass** die Auswertungseinrichtung (4) dazu eingerichtet ist, in einem aktuellen Einzelbild der Bildsequenz eine Position und/oder eine Größe des applizierten Behandlungsmediums (P) zu bestimmen.

6. Vorrichtung (1) nach einem der Ansprüche 3 bis 5, **dadurch gekennzeichnet, dass** die Auswertungseinrichtung (4) dazu eingerichtet ist, in dem aktuellen Einzelbild der Bildsequenz eine Position, eine Ausrichtung und/oder eine Abmessung des Kopfs (6) des Instruments (2) zu bestimmen.

7. Vorrichtung (1) nach einem der vorhergehenden Ansprüche, **dadurch gekennzeichnet, dass** die Auswertungseinrichtung (4) dazu eingerichtet ist, das Behandlungsmedium (P) in den Daten der Bilderfassungseinrichtung (3) anhand eines Farb- und/oder Formmerkmals zu erkennen, vorzugsweise zumindest anhand eines der folgenden Merkmale: einem Farbwertebereich, einem Helligkeitswertebereich und einem Sättigungswertebereich.

8. Vorrichtung (1) nach einem der vorhergehenden Ansprüche, **dadurch gekennzeichnet, dass** die Auswertungseinrichtung (4) dazu eingerichtet ist, den Kopf (6) in den Daten der Bilderfassungseinrichtung (3) anhand eines Farb- und/oder Formmerkmals oder anhand eines auf dem Kopf (6) angebrachten Markers zu erkennen.

9. Vorrichtung (1) nach einem der vorhergehenden Ansprüche, **dadurch gekennzeichnet, dass** die Auswertungseinrichtung (4) dazu eingerichtet ist, in dem aktuellen Einzelbild mittels einer Suchroutine in einem Suchbereich (S) das Behandlungsmedium (P) zu erkennen, wobei der Suchbereich für das Behandlungsmedium (P) anhand der Position, der Ausrichtung und/oder der Abmessung des Kopfes (6) verändert wird.

10. Vorrichtung (1) nach einem der vorhergehenden Ansprüche, insbesondere nach Anspruch 9, **dadurch gekennzeichnet, dass** die Auswertungseinheit (4) dazu eingerichtet ist, eine prädizierte Position (Pos*_t) des Behandlungsmediums (P) im aktuellen Einzelbild anhand einer Position (Pos_t-1) und/oder Geschwindigkeit (V_t-1) des Behandlungsmediums (P) aus einem vorherigen Einzelbild zu bestimmen,
wobei vorzugsweise der Suchbereich (S), in welchem die Suchroutine in dem aktuellen Einzelbild das Behandlungsmedium (P) erkennt, anhand der prädizierten Position (Pos*_t) und/oder der Geschwindigkeit (V_t-1) verändert wird.

11. Vorrichtung (1) nach einem der vorhergehenden Ansprüche, **dadurch gekennzeichnet, dass** die Auswertungseinrichtung (4) dazu eingerichtet ist, dass der zu behandelnden Bereich (B) vor dem Applizieren des Behandlungsmediums (P) von dem Anwender festlegbar ist.

12. Vorrichtung (1) nach einem der vorhergehenden Ansprüche, **dadurch gekennzeichnet, dass** die Auswertungseinrichtung (4) dazu eingerichtet ist, in den Daten der Bilderfassungseinrichtung (3) charakteristische Bildmerkmale des zu behandelnden Bereichs (B) oder in den Bereich (B) der Gewebeoberfläche aufgetragene Referenzmarker zu erkennen und die Behandlungsspur (T) dem zu behandelnden Bereich (B) ortsgetreu zuzuordnen.

13. Vorrichtung (1) nach einem der vorhergehenden Ansprüche, **dadurch gekennzeichnet, dass** die Auswertungseinrichtung (4) dazu eingerichtet ist, das Applizieren des Behandlungsmediums (P) durch das Instrument (2) in Abhängigkeit von der lokalen Dosierung zu steuern.

14. Verfahren zur bildgestützten Unterstützung eines Anwenders, umfassend:
- Erfassen (V1) mittels einer Bilderfassungseinrichtung (3) eine Bildsequenz eines zu behandelnden Bereichs (B), in welchem mit einem Instrument (2) ein Behandlungsmedium (P) an veränderbaren Orten applizierbar ist, wobei das applizierbare Behandlungsmedium (P) ein Plasma ist;
- Erkennen mittels einer Auswertungseinrichtung (4) des Behandlungsmediums (P) und/oder eines Kopfes (6) des Instruments (2) in der Bildsequenz des zu behandelnden Bereichs (B);
- Bestimmen (V2) mittels der Auswertungseinrichtung (4) einer Behandlungsspur (T) des Behandlungsmediums (P) relativ zum Bereich (B);
- Ermitteln (V3) mittels der Auswertungseinrichtung (4) einer ortsaufgelösten Dosierung (D) für den zu behandelnden Bereich (B) anhand der Behandlungsspur (T) und Messparametern, die von einer Versorgungseinheit (9) des Instruments (2) über eine kommunikative Verbindung an die Auswertungseinrichtung (4) übermittelt werden; und
- Darstellen (V4) mittels einer Wiedergabeeinrichtung (5, 5a, 5b, 5c) der Behandlungsspur und/oder der ortsaufgelösten Dosierung (D) in Bezug auf den zu behandelnden Bereich (B).

## Claims

1. Device (1) for image based support of a user comprising:
- an instrument (2) that is configured to apply a treatment medium (P) at alterable locations in an area (B) to be treated, wherein the treatment medium (P) that can be applied is a plasma;
- an image capture device (3) that is configured to capture the area (B) to be treated;
- an evaluation device (4) that is configured to determine a treatment trace (T) of the treatment medium (P) relative to the area (B) to be treated from data of the image capture device (3) and to determine a spatially resolved dosage (D) for the area (B) to be treated based on the treatment trace (T); as well as
- a representation device (5, 5a, 5b, 5c) that is configured to optically display the treatment trace (T) and/or the spatially resolved dosage (D) in relation to the area (B) to be treated for the user,
wherein the evaluation device (4) is configured to recognize the treatment medium (P) and/or a head (6) of the instrument (2) in an image sequence,
**characterized in that** the evaluation device (4) is communica-tively connected with a supply unit (9) of the instrument (2),
whereby the evaluation device (4) is configured to determine the spatially resolved dosage (D) in addition based on measurement parameters that are transmitted by the supply unit (9) to the evaluation device (4).

2. Device (1) according to claim 1, **characterized in that** the treatment medium (P) that can be applied is a low- or non-thermal plasma.

3. Device (1) according to claim 1 or 2, **characterized in that** the image capture device (3) is configured to capture the area (B) to be treated by an image sequence comprising a multiplicity of individual images.

4. Device (1) according to any of the preceding claims **characterized in that** the evaluation device (4) is configured to trace the treatment medium (P) and/or a head (6) of the instrument (2) over multiple individual images in the image sequence.

5. Device (1) according to claim 3 or 4, **characterized in that** the evaluation device (4) is configured to determine a position and/or a size of the applied treatment medium (P) in a current individual image of the image sequence.

6. Device (1) according to any of the claims 3 to 5, **characterized in that** the evaluation device (4) is configured to determine a position, an orientation and/or a dimension of the head (6) of the instrument (2) in the current individual image of the image sequence.

7. Device (1) according to any of the preceding claims, **characterized in that** the evaluation device (4) is configured to recognize the treatment medium (P) in data of the image capture device (3) based on a color and/or shape feature or based on a marker attached on the head (6).

8. Device (1) according to any of the preceding claims, **characterized in that** the evaluation device (4) is configured to recognize the head (6) in data of the image capture device (3) based on a color and/or shape feature or based on a marker attached on the head (6).

9. Device (1) according to any of the preceding claims, **characterized in that** the evaluation device (4) is configured to recognize the treatment medium (P) in a search area (S) in the current individual image by means of a search routine, whereby the search area for the treatment medium (P) is modified based on the position, the orientation and/or the dimension of the head (6).

10. Device (1) according to any of the preceding claims, particularly according to claim 9, **characterized in that** the evaluation device (4) is configured to determine a predicted position (Pos*_t) of the treatment medium (P) in the current individual image based on a position (Pos_t-1) and/or velocity (V_t-1) of the treatment medium (P) from a previous individual image, wherein preferably the search area (S), in which the search routine recognizes the treatment medium (P) in the current individual image, is modified based on the predicted position (Pos*_t) and/or the velocity (V_t-1).

11. Device (1) according to any of the preceding claims, **characterized in that** the evaluation device (4) is configured so that the area (B) to be treated can be defined by the user prior to the application of treatment medium (P).

12. Device (1) according to any of the preceding claims, **characterized in that** the evaluation device (4) is configured to recognize characteristic image features of the area (B) to be treated in data of the image capture device (3) or to recognize reference markers applied in the area (B) of the tissue surface and to assign the treatment trace (T) to the area (B) to be treated in a spatially accurate manner.

13. Device (1) according to any of the preceding claims, **characterized in that** the evaluation device (4) is configured to control the application of the treatment medium (P) by the instrument (2) depending on the local dosage.

14. Method for image based support of a user, comprising:
- Capturing (V1) by means of an image capturing device (3) an image sequence of an area (B) to be treated in which a treatment medium (P) can be applied at alterable locations, wherein the treatment medium (P) that can be applied is a plasma;
- Recognizing by means of an evaluation device (4) the treatment medium (P) and/or a head (6) of the instrument (2) in the image sequence of the area (B) to be treated,
- Determining (V2) by means of the evaluation device (4) a treatment trace (T) of the treatment medium (P) relative to the area (B) to be treated;
- Determining (V3) by means of the evaluation device (4) a spatially resolved dosage (D) for the area (B) to be treated based on the treatment trace (T) and measurement parameters that are transmitted by a supply unit (9) of the instrument (2) to the evaluation device (4) via a communication connection;
- Representing (V4) by means of a representation device (5, 5a, 5b, 5c) the treatment trace and/or the spatially resolved dosage (D) with reference to the area (B) to be treated.

## Revendications

1. Dispositif (1) d'aide assistée par image à un utilisateur, comprenant :
- un instrument (2) qui est agencé pour appliquer un fluide de traitement (P) à des endroits variables dans une zone à traiter (B), le fluide de traitement (P) qui peut être appliqué étant un plasma ;
- un dispositif d'acquisition d'images (3) qui est conçu pour détecter la zone à traiter (B) ;
- un dispositif d'analyse (4) qui est conçu
pour définir une trace de traitement (T) du fluide de traitement (P) par rapport à la zone à traiter (B), à partir des données du dispositif d'acquisition d'image (3), et pour déterminer un dosage à résolution spatiale (D) pour la zone à traiter (B), à l'aide de la trace de traitement (T) ; ainsi que
- un dispositif de reproduction (5, 5a, 5b, 5c) qui est conçu pour représenter optiquement pour l'utilisateur la trace de traitement (T) et/ou le dosage à résolution spatiale (D) par rapport à la zone à traiter (B),
le dispositif d'analyse (4) étant conçu pour identifier le fluide de traitement (P) et/ou une tête (6) de l'instrument (2) dans une séquence d'images de la zone à traiter (B), **caractérisé en ce que**
le dispositif d'analyse (4) est relié sur le plan de la communication à une unité d'alimentation (9) de l'instrument (2),
le dispositif d'analyse (4) étant conçu pour réaliser la détermination du dosage à résolution spatiale (D) en plus sur la base de paramètres de mesure qui sont transmis au dispositif d'analyse (4) par l'unité d'alimentation (9).

2. Dispositif (1) selon la revendication 1, **caractérisé en ce que** le fluide de traitement (P) applicable est un plasma faiblement thermique ou non thermique.

3. Dispositif (1) selon la revendication 1 ou 2, **caractérisé en ce que** le dispositif d'acquisition d'images (3) est conçu pour capturer la zone à traiter (B) dans la séquence d'images comportant une pluralité d'images individuelles.

4. Dispositif (1) selon une des revendications précédentes, **caractérisé en ce que** le dispositif d'analyse (4) est conçu pour suivre le fluide de traitement (P) et/ou la tête (6) de l'instrument (2) sur plusieurs images individuelles de la séquence d'images.

5. Dispositif (1) selon la revendication 3 ou 4, **caractérisé en ce que** le dispositif d'analyse (4) est conçu pour déterminer, dans une image individuelle actuelle de la séquence d'images, une position et/ou une dimension du fluide de traitement (P) appliqué.

6. Dispositif (1) selon une des revendications 3 à 5, **caractérisé en ce que** le dispositif d'analyse (4) est conçu pour déterminer, dans l'image individuelle actuelle de la séquence d'images, une position, une orientation et/ou une dimension de la tête (6) de l'instrument (2).

7. Dispositif (1) selon une des revendications précédentes, **caractérisé en ce que** le dispositif d'analyse (4) est conçu pour identifier le fluide de traitement (P) dans les données du dispositif d'acquisition d'images (3), à l'aide d'une caractéristique de couleur et/ou de forme, de préférence au moins à l'aide de l'une des caractéristiques suivantes : une gamme de valeurs chromatiques, une gamme de valeurs de luminosité et une gamme de valeurs de saturation.

8. Dispositif (1) selon une des revendications précédentes, **caractérisé en ce que** le dispositif d'analyse (4) est conçu pour identifier la tête (6) dans les données du dispositif d'acquisition d'images (3), à l'aide d'une caractéristique de couleur et/ou de forme ou à l'aide d'un marqueur placé sur la tête (6).

9. Dispositif (1) selon une des revendications précédentes, **caractérisé en ce que** le dispositif d'analyse (4) est conçu pour identifier, dans l'image individuelle actuelle, au moyen d'une routine de recherche, le fluide de traitement (P) dans une zone de recherche (S), ladite zone de recherche pour le fluide de traitement (P) étant modifiée à l'aide de la position, de l'orientation et/ou de la dimension de la tête (6).

10. Dispositif (1) selon une des revendications précédentes, en particulier selon la revendication 9, **caractérisé en ce que** l'unité d'analyse (4) est conçue pour déterminer une position prédite (Pos*_t) du fluide de traitement (P) dans l'image individuelle actuelle, à l'aide d'une position (Pos_t-1) et/ou d'une vitesse (V_t-1) du fluide de traitement (P), à partir d'une image individuelle précédente,
la zone de recherche (S), dans laquelle la routine de recherche identifie le fluide de traitement (P) dans l'image individuelle actuelle, étant de préférence modifiée à l'aide de la position prédite (Pos*_t) et/ou de la vitesse (V_t-1).

11. Dispositif (1) selon une des revendications précédentes, **caractérisé en ce que** le dispositif d'analyse (4) est conçu pour que le zone à traiter (B) puisse être définie par l'utilisateur avant l'application du fluide de traitement (P).

12. Dispositif (1) selon une des revendications précédentes, **caractérisé en ce que** le dispositif d'analyse (4) est conçu pour identifier, dans les données du dispositif d'acquisition d'images (3), des propriétés d'image caractéristiques de la zone à traiter (B) ou bien des marqueurs de référence appliqués dans la zone (B) de la surface des tissus, et pour associer la trace de traitement (T) fidèlement à l'emplacement de la zone à traiter (B).

13. Dispositif (1) selon une des revendications précédentes, **caractérisé en ce que** le dispositif d'analyse (4) est conçu pour contrôler l'application du fluide de traitement (P) par l'instrument (2), en fonction du dosage local.

14. Procédé d'aide assistée par images pour un utilisateur, comprenant :
- la capture (V1), au moyen d'un dispositif d'acquisition d'images (3), d'une séquence d'images d'une zone à traiter (B) dans laquelle un fluide de traitement (P) peut être appliqué avec un instrument (2) à des endroits variables, le fluide de traitement (P) applicable étant un plasma ;
- identification, au moyen d'un dispositif d'analyse (4), du fluide de traitement (P) et/ou d'une tête (6) de l'instrument (2) dans la séquence d'images de la zone à traiter (B) ;
- définition (V2), au moyen du dispositif d'analyse (4), d'une trace de traitement (T) du fluide de traitement (P) par rapport à la zone (B) ;
- détermination (V3), au moyen du dispositif d'analyse (4), d'un dosage à résolution spatiale (D) pour la zone à traiter (B), à l'aide de la trace de traitement (T) et de paramètres de mesure qui sont transmis au dispositif d'analyse (4) par une unité d'alimentation (9) de l'instrument (2), via une liaison de communication ; et
- représentation (V4), au moyen d'un dispositif de reproduction (5, 5a, 5b, 5c), de la trace de traitement et/ou du dosage à résolution spatiale (D), par rapport à la zone à traiter (B).
